# FASCICULE DE BREVET EUROPEEN

(11) **EP 2 774 601 B1**
(45) Date de publication et mention de la délivrance du brevet: **16.08.2017**
(21) Numéro de dépôt: 14157674.4
(22) Date de dépôt: 04.03.2014
(51) Int. Cl.: A61K 8/31, A61K 8/33, A61K 8/34, A61K 8/36, A61K 8/37, A61Q 19/00, A61Q 19/08, A61K 8/97

(54) **Produit cosmétique et/ou dermatologique à base d'un extrait de liège, d'au moins une partie de l'arbre fournissant le liège, et d'au moins un corps gras naturel, son procédé de préparation et composition le contenant**
Kosmetik- und/oder Hautpflegeprodukt auf der Basis von Korkeichenextrakt, mindestens eines Teils des Baums, der den Kork produziert, und mindestens eines natürlichen Fettkörpers, sein Herstellungsverfahren und die Zusammensetzung, die es enthält
Cosmetic and/or dermatological product made of cork extract, from at least one portion of the tree that provides cork, and at least one natural greasy substance, method for preparing same and composition containing same

(30) Priorité: 06.03.2013 FR 1352023
(43) Date de publication de la demande: 10.09.2014
(73) Titulaire: Oleos, 34400 Lunel (FR); DIAM BOUCHAGE, 66400 Ceret (FR)
(72) Inventeur: Balbusquier, Clothilde, 66160 LE BOULOU (FR); Lemoine, Noémie, 34400 Lunel (FR); Loisel, Christophe, 66180 VILLENEUVE DE LA RAHO (FR); Rossignol-Castera, Anne, 34160 RESTINCLIERES (FR); Tourneix, Dominique, 66240 SAINT ESTEVE (FR)
(74) Mandataire: Casalonga

(56) Documents cités:
- WO-A1-01/23155
- FR-A1- 2 841 133
- FR-A1- 2 943 684
- DATABASE WPI Week 201060 Thomson Scientific, London, GB; AN 2010-J99187 XP2715972, & CN 101 756 834 A (FAN K) 30 juin 2010 (2010-06-30)
- DATABASE WPI Week 201057 Thomson Scientific, London, GB; AN 2010-J99192 XP2715973, & CN 101 756 830 A (CHEN J) 30 juin 2010 (2010-06-30)

## Description

La présente invention concerne un produit cosmétique et/ou dermatologique à base d'un extrait de liège, d'au moins une partie de l'arbre fournissant le liège choisie parmi ses feuilles, ses fleurs, ses racines, ses bourgeons et leurs mélanges, de préférence parmi ses feuilles, ses fleurs et leurs mélanges, et d'au moins un corps gras naturel.

L'invention concerne aussi son procédé de préparation et une composition cosmétique et/ou dermatologique, ainsi que son utilisation dans la prévention du vieillissement de la peau ou pour la protection de la peau vis-à-vis des rayons ultraviolets ou dans le traitement des érythèmes des peaux sensibles ou réactives, ou encore dans le traitement des érythèmes solaires.

Le liège est un matériau naturel, imperméable et léger, qui provient de l'écorce de certaines espèces de chênes, tels que le chêne-liège et plus particulièrement l'espèce *Quercus suber,* qui se rencontrent, pour la plupart, dans les pays du pourtour méditerranéen, en Europe et au Maghreb.

En particulier, on récolte traditionnellement l'écorce de l'arbre de l'espèce *Quercus suber* et on la traite afin de produire le liège destiné à être utilisé dans l'industrie des bouchons. Le liège est principalement constitué de subérine (33 à 50 % en poids) et en moindre part de lignine (12 à 30% en poids), de polysaccharides (6 à 25 % à en poids), de produits extractibles (8 à 24% en poids) comme les cires, les tanins et des composés volatiles, et de composés minéraux (2 à 4% en poids).

Le traitement de ce composé naturel par un fluide supercritique avec le procédé décrit dans le brevet européen No. 1 216 123, conduit à l'élimination de certains composés organiques responsables du goût de bouchon. Ce procédé permet ainsi un entrainement sélectif de certains composés organiques extractibles qui se présentent au final sous la forme d'un mélange de deux phases : un surnageant graisseux ou phase grasse, et une phase aqueuse. Ce mélange sera dénommé ci-après extrait de liège.

La demanderesse a trouvé de manière surprenante que cette phase grasse associée à au moins une partie de l'arbre fournissant le liège, choisie parmi ses feuilles, ses fleurs, ses racines, ses bourgeons et leurs mélanges, puis traitée avec un procédé particulier tel que décrit dans la demande de brevet français No. 2 943 684, en présence d'au moins un corps gras naturel conduit à un produit cosmétique et/ou dermatologique tel que décrit ci-dessous ayant des propriétés cosmétiques et/ou dermatologiques améliorées telles qu'une hydratation de la peau, ou permettant de prévenir l'apparition des signes du vieillissement de la peau.

Le vieillissement de la peau s'accompagne d'un certain nombre de signes, et en particulier de la formation de rides plus ou moins étendues, outre une perte d'élasticité et un amincissement.

Pour ralentir l'apparition de ces signes, il faut protéger, hydrater et nourrir la peau en intervenant sur tous les mécanismes biologiques et cellulaires qui maintiennent l'équilibre et la cohésion de l'épiderme.

Le produit selon l'invention permet de limiter l'apparition de ces signes. Il a été trouvé que le produit selon l'invention présente une action anti-âge par prévention du stress oxydant couplée à un effet hydratant. Le produit présente une capacité à piéger les radicaux libres.

Le vieillissement cutané résulte d'un certain nombre d'altérations qui surviennent au cours du temps mais qui peuvent être également induites par des facteurs externes comme les rayonnements ultraviolets et la pollution.

Il a été trouvé qu'il permettait notamment de protéger la peau contre les rayonnements ultraviolets.

Par ailleurs, il présente également une action anti-érythème pour le traitement préventif et apaisant des rougeurs propres aux peaux sensibles ou réactives, et permet aussi de traiter les érythèmes solaires. En particulier, même utilisé à faible dose soit moins de 3 % dans un produit cosmétique, il permet de traiter les érythèmes tout en préservant l'intégrité et la souplesse de la peau.

L'invention a donc pour objet un produit cosmétique et/ou dermatologique comprenant :
0,5 à 10 % en poids, de préférence de 1 à 7 % en poids d'acides gras en C₆₋₃₀, de préférence en C₆₋₂₄, saturés ou insaturés, linéaires ou ramifiés,
3 à 15 % en poids, de préférence de 5 à 10 % en poids d'alcools gras en C₆₋₃₀, saturés ou insaturés, linéaires ou ramifiés,
0,5 à 10 % en poids, de préférence de 1 à 5 % en poids d'un ou plusieurs phytostérols,
0,1 à 10 % en poids, de préférence de 0,5 à 5 % en poids d'un ou plusieurs terpènes différents du squalène,
1 à 15 % en poids, de préférence de 3 à 10 % en poids de squalène, et 50 à 90 % en poids, de préférence de 55 à 85 % en poids de triglycérides,
par rapport au poids total du produit.

L'invention a encore pour objet son procédé de préparation à partir de liège et d'au moins une partie de l'arbre fournissant le liège choisie parmi ses feuilles, ses fleurs, ses racines, ses bourgeons et leurs mélanges, de préférence parmi ses feuilles, ses fleurs et leurs mélanges, comprenant les étapes qui consistent à :
a) mettre le liège en contact avec du CO₂ supercritique, à une température allant de 10 à 120 °C et sous une pression allant de 10 à 600 bars, éventuellement en présence d'un co-solvant,
b) récupérer l'extrait de liège entrainé par le CO₂ supercritique, sous la forme d'un mélange de phases aqueuse et grasse,
c) séparer la phase grasse de l'extrait de liège de la phase aqueuse,
d) mélanger ladite phase grasse de l'extrait de liège avec une ou des parties de l'arbre fournissant le liège réduite(s) en poudre, et un ou plusieurs corps gras naturels, à une température supérieure aux températures de fusion de la phase grasse et du ou des corps gras naturels, sous une atmosphère dépourvue ou essentiellement dépourvue d'O₂,
e) chauffer le mélange obtenu, à une température allant de 100 à 140 °C, de préférence de 110 à 130 °C, pendant une durée inférieure à 10 minutes, de préférence inférieure à 5 minutes, sous une atmosphère dépourvue ou essentiellement dépourvue d'O₂,
f) effectuer une microdispersion de ladite phase grasse de l'extrait de liège, d'une ou des parties de l'arbre fournissant le liège réduite(s) en poudre dans le ou les corps gras naturels, à une température supérieure aux températures de fusion de la phase grasse et du ou des corps gras naturels, sous une atmosphère dépourvue ou essentiellement dépourvue d'O₂, et à
g) éventuellement répéter les deux étapes e) et f).

Par opérer « sous atmosphère dépourvue ou essentiellement dépourvue d'O₂ », on entend opérer avec un gaz ou atmosphère inerte, sous vide ou vide partiel. La teneur résiduelle en oxygène doit être suffisamment basse pour ne pas provoquer de réactions d'oxydation sensibles à la température du traitement thermique. Cette teneur résiduelle, mesurée par un détecteur d'oxygène ou une sonde à oxygène, est de préférence inférieure à 3 % en poids. On peut utiliser une atmosphère inerte, par exemple de l'azote et de préférence un balayage continu d'azote, permettant l'extraction de l'oxygène présent ou susceptible de se former. On peut ainsi, par exemple, employer un réacteur clos avec une extraction continue de l'oxygène par flux d'azote. On peut aussi faire un barbotage d'azote, associé au flux d'azote, tout au moins au début du traitement thermique. On peut aussi conduire ces étapes sous vide.

Préférentiellement, les étapes d) à f) sont effectuées sous une atmosphère saturée d'azote.

Un autre objet de l'invention concerne le produit susceptible d'être obtenu par le procédé tel que décrit ci-dessus, qui se trouve sous une forme semi-solide à température ambiante (20-25 °C). Ce produit se présente comme un beurre gras, pâteux épais et homogène à l'oeil c'est-à-dire sous une seule phase et sans cristaux visibles. Il fond à une température de l'ordre de 30°C.

L'invention a encore pour objet une composition cosmétique et/ou dermatologique comprenant le produit selon l'invention, dans un milieu cosmétiquement ou dermatologiquement acceptable.

L'invention a encore pour objet un procédé de traitement cosmétique des signes du vieillissement de la peau mettant en oeuvre la composition selon l'invention.

Un autre objet consiste en l'utilisation du produit selon l'invention en cosmétique, pour hydrater les couches supérieures de la peau.

Un autre objet de l'invention concerne l'utilisation du produit comme médicament, et plus particulièrement dans le traitement des érythèmes des peaux sensibles et réactives.

L'invention concerne également son utilisation pour la photoprotection de la peau ou encore dans le traitement des érythèmes solaires.

D'autres objets, caractéristiques, aspects et avantages de l'invention apparaîtront encore plus clairement à la lecture de la description et de l'exemple qui suit.

Dans ce qui va suivre, et à moins d'une autre indication, les bornes d'un domaine de valeurs sont comprises dans ce domaine, notamment dans les expressions « compris entre » et « allant de ... à ... ».

Par ailleurs, l'expression « au moins un » utilisée dans la présente description est équivalente à l'expression « un ou plusieurs ».

Le produit cosmétique et/ou dermatologique selon l'invention comprend :
0,5 à 10 % en poids, de préférence de 1 à 7 % en poids d'acides gras en C₆₋₃₀, de préférence en C₆₋₂₄, saturés ou insaturés, linéaires ou ramifiés,
3 à 15 % en poids, de préférence de 5 à 10 % en poids d'alcools gras en C₆₋₃₀, saturés ou insaturés, linéaires ou ramifiés,
0,5 à 10 % en poids, de préférence de 1 à 5 % en poids d'un ou plusieurs phytostérols,
0,1 à 10 % en poids, de préférence de 0,5 à 5 % en poids d'un ou plusieurs terpènes différents du squalène,
1 à 15 % en poids, de préférence de 3 à 10 % en poids de squalène, et 50 à 90 % en poids, de préférence de 55 à 85 % en poids de triglycérides,
par rapport au poids total du produit.

Les acides gras en C₆₋₃₀ sont de préférence linéaires et saturés, mono-insaturés ou di-insaturés, et peuvent être choisis parmi l'acide hexanoïque (ou caproïque), l'acide octanoïque (ou caprylique), l'acide décanoïque (ou caprique), l'acide laurique, l'acide myristique, l'acide palmitique, l'acide margarique, l'acide stéarique, l'acide arachidique, l'acide béhénique, l'acide lignocérique, l'acide palmitoléique, l'acide oléique, l'acide linoléique, et leurs mélanges.

Dans un mode de réalisation particulier, les acides gras en C₆₋₃₀ sont sous la forme d'un mélange de :
0,2 à 1,5 % en poids d'acide hexanoïque (ou caproïque),
4 à 20 % en poids d'acide octanoïque (ou caprylique),
2 à 15 % en poids d'acide décanoïque (ou caprique),
30 à 60 % en poids d'acide laurique,
10 à 30 % en poids d'acide myristique,
4 à 12 % en poids d'acide palmitique,
1 à 6 % en poids d'acide stéarique,
1 à 11 % en poids d'acide oléique,
0,5 à 4 % en poids d'acide linoléique et
moins de 3 % en poids d'acide gras en C₂₀₋₃₀,
par rapport au poids total du mélange d'acides gras en C₆₋₃₀.

A titre d'exemples d'alcool gras en C₆₋₃₀, on peut notamment citer l'octadécanol, l'eicosanol, le docosanol et l'eicosènol.

A titre d'exemples de phytostérols, on peut notamment citer le β-sitostérol, le stigmastanol, le campestérol et l'avénastérol.

A titre d'exemples de terpènes différents du squalène, on peut notamment citer la friedéline, la bétuline, le lupen-3-one, la sitost-4-én-3-one, et les alcools triterpéniques comme le lupéol, le bétulinol, l'α-amyrine et l'érythrodiol.

De préférence, le produit cosmétique et/ou dermatologique contient plus de 20 % en poids, mieux de 20 à 50 % en poids, de composés terpéniques incluant le squalène, les terpènes différents du squalène et les phytostérols.

Les triglycérides sont des esters de glycérol et d'acides gras en C₄₋₃₀, de préférence en C₈₋₁₈, notamment la tristéarine, la trioléine et la trilinoléine.

Le produit selon l'invention présente de préférence un taux d'insaponifiable allant de 12 à 26 %, mieux de 15 à 22 %. Ce taux peut être mesuré selon la norme NF EN ISO 3596.

Selon un mode de réalisation, le produit selon l'invention présente une teneur en eau et en volatils telle que mesurée, par exemple selon la norme NF EN ISO 662, inférieure à 3 %, mieux allant de 0 à 1,5 %.

Dans un autre mode de réalisation, le produit selon l'invention présente une acidité oléique telle que mesurée, par exemple selon la norme NF EN ISO 660, inférieure à 12 %, mieux allant de 0,5 à 6 %.

De préférence, le produit selon l'invention présente un indice de peroxyde inférieur à 20 méq O₂/kg, mieux inférieur à 10 méq O₂/kg et préférentiellement inférieur à 5 méq O₂/kg. Cet indice de peroxyde peut être mesuré, par exemple selon la norme NF EN ISO 3960.

Le produit selon l'invention est préparé selon le procédé mentionné ci-dessus et décrit plus en détail ci-dessous. Il met en oeuvre des matières premières naturelles et aucun solvant organique comme l'hexane ou le dichlorométhane. Ce procédé permet d'obtenir un rendement d'extraction élevé de molécules actives, ayant un effet antioxydant notamment, de conserver les propriétés bénéfiques de toutes les matières premières végétales et d'obtenir un effet synergique par rapport aux matières premières seules.

Le procédé de préparation selon l'invention met en oeuvre du liège provenant notamment de chêne-liège comme le Quercus Suber, et une ou plusieurs parties de l'arbre fournissant le liège choisies parmi ses feuilles, ses fleurs, ses racines, ses bourgeons et leurs mélanges, de préférence parmi ses feuilles, ses fleurs et leurs mélanges, encore mieux parmi le mélange de feuilles et de fleurs. Il comprend les étapes a) à f) décrites généralement ci-dessus.

Selon l'invention, l'étape a) s'effectue à une température allant de 10 à 120 °C, de préférence de 40 à 80 °C, sous une pression de 1 à 60 MPa (10 à 600 bars), mieux de 10 à 30 MPa (100 à 300 bars), éventuellement en présence d'un co-solvant. Le co-solvant peut être choisi, par exemple, parmi l'eau et les solutions aqueuses. A titre d'exemples de solutions aqueuses, on peut notamment citer les mélanges d'eau et d'alcools en C₁₋₄ comme le méthanol, l'éthanol, l'isopropanol et le butanol, et plus particulièrement l'éthanol. Le co-solvant peut être utilisé en une quantité allant de 0,01 à 10 % en poids, de préférence de 0,02 à 1 % en poids par rapport au poids de CO₂ supercritique utilisé.

Cette étape a) correspondant à un traitement au CO₂ supercritique est décrite plus en détail dans le brevet EP 1 216 123.

Le traitement du liège par le CO₂ supercritique permet un entrainement sélectif de certains composés organiques, ces composés organiques extraits constituant ainsi l'extrait de liège. Ce dernier se présente sous la forme d'un mélange de deux phases : un surnageant graisseux qui constitue la phase grasse et une phase aqueuse.

La phase grasse de l'extrait de liège récupérée à l'étape b), est ensuite séparée de la phase aqueuse dans l'étape c), notamment par simple décantation statique ou centrifugation. La phase grasse séparée se présente en particulier sous la forme d'une masse grasse solide à température ambiante (20-25°C). Elle a un aspect visuel cireux et un aspect hétérogène, c'est-à-dire comprenant des amas cristallisés blanchâtres visibles. De l'eau résiduelle est généralement présente en une teneur allant de 10 à 40 % en poids par rapport au poids total de la phase grasse.

Dans un mode de réalisation particulier de l'invention, on peut traiter cette phase grasse avant l'étape d), pour l'homogénéiser, i.e. éliminer les cristaux blanchâtres, et la sécher, par exemple de la manière suivante : on réalise un chauffage sous agitation pour faire fondre la phase grasse, c'est-à-dire à une température supérieure au point de fusion de la phase grasse, qui va de préférence de 40°C à 90 °C, mieux de 50°C à 60°C. Cette opération est préférentiellement conduite dans un réacteur inox à double enveloppe et sous atmosphère saturée d'azote pour limiter les risques d'oxydation. Le chauffage et le mélange doivent être conduits notamment jusqu'à obtention d'un produit homogène et liquéfié, dit phase grasse d'extrait de liège traitée. La durée de cette opération varie par exemple de 15 min. à 60 min. selon le matériel utilisé. Cette opération a également pour effet l'élimination de l'eau résiduelle présente dans la phase grasse.

La phase grasse d'extrait de liège traitée se présente comme une masse grasse homogène, solide à température ambiante. Elle a un aspect visuel cireux et une couleur jaune beige. Sa teneur en eau est de préférence inférieure à 5 % en poids par rapport au poids total de la phase grasse traitée. Son niveau d'oxydation évalué par l'indice de peroxyde est de préférence inférieur à 5 méq O₂/kg.

La phase grasse de l'extrait de liège contient les composés du liège brut extractibles par le gaz carbonique à l'état supercritique, à savoir des composés triterpéniques, tels que le squalène, la friedéline, le lupéol, la sitosténone et l'acide bétulinique, et ses dérivés comme la bétuline ; des acides gras en C₁₆₋₃₀, saturés ou insaturés, comme l'acide linoléique (ou octadécadiénoïque), l'acide palmitique, l'acide oléique, l'acide stéarique, l'acide arachidique (ou eicosanoïque), l'acide béhénique et l'acide lignocérique ; des alcools gras en C₁₈₋₃₀, saturés ou insaturés, comme l'octadécanol, l'eicosanol, le docosanol, l'eicosènol, le docosènol, l'heneicosanol, le tricosanol, le tétracosanol et l'hexacosanol ; les esters gras d'acide acétique et d'alcools gras en C₁₆₋₃₀, comme les acétates d'octadécyle et de docosyle ; les phytostérols comme le sitostérol ; ou autres hydrocarbures.

Les composés plus polaires co-entraînés au cours de l'extraction CO₂ supercritique par la présence d'eau ou d'un co-solvant alcoolique, restent préférentiellement dans la phase aqueuse de l'extrait brut. Certains composés de polarité intermédiaire tels que les phénols volatils et non volatils (acides phénoliques, polyphénols..) se partagent entre la phase aqueuse et le surnageant gras de l'extrait brut.

La phase grasse éventuellement traitée est utilisée dans l'étape d) à raison de 20 à 40 % en poids, de préférence de 25 à 35 % en poids par rapport au poids total du mélange.

La phase grasse éventuellement traitée est ensuite mélangée avec une ou des parties de l'arbre fournissant le liège réduite(s) en poudre, dans l'étape d). Ces parties de l'arbre fournissant le liège sont choisies parmi ses feuilles, ses fleurs, ses racines, ses bourgeons et leurs mélanges, plus particulièrement parmi ses feuilles, ses fleurs et leurs mélanges, et mieux encore sont un mélange de ses feuilles et de ses fleurs.

De préférence, les feuilles du chêne-liège sont récoltées au moment de la floraison. En particulier, les feuilles sont de forme irrégulière et d'aspect cireux. Les fleurs très petites, de couleur jaune clair, sont organisées en grappes d'aspect très poudreux.

Avant leur utilisation dans le procédé selon l'invention, et plus particulièrement dans l'étape d) du procédé, ces parties de l'arbre fournissant le liège sont réduites en poudre, par exemple, de la manière suivante : dans un premier temps, elles sont récoltées puis déshydratées à l'air au moment de la récolte. On les place ensuite dans un séchoir à air chaud à une température inférieure à 40 °C, de préférence allant de 25 à 40 °C, mieux de 30 à 35 °C de manière à obtenir une teneur en eau résiduelle comprise entre 5 et 15 % en poids, de préférence entre 8 et 10 % en poids par rapport au poids total du mélange obtenu.

Pour obtenir cette teneur en eau résiduelle, il est aussi possible de laisser sécher les parties de l'arbre que l'on veut utiliser dans l'étape d), à température ambiante pendant plusieurs jours.

Dans un deuxième temps, les parties de l'arbre fournissant le liège déshydratées sont broyées, par exemple, dans un broyeur à couteaux. Le broyat est ensuite pulvérisé notamment dans un malaxeur à hélices, à une température allant de préférence de -18 à -80 °C, mieux -50 à -80 °C. On obtient alors une poudre fine et régulière de granulométrie allant en particulier de 100 µm à 1 mm, de préférence de 200 à 400 µm.

La quantité de poudre d'une partie de l'arbre fournissant le liège utilisée dans l'étape d) va de préférence de 0,01 à 15 % en poids, de préférence de 0,1 à 10 % en poids, mieux 1 à 10 % en poids par rapport au poids total du mélange.

Les corps gras naturels utilisés dans l'invention, peuvent être une huile ou un beurre végétal qui peut être raffiné, désodorisé ou vierge. On peut citer notamment l'huile de colza, l'huile de tournesol, l'huile de soja, l'huile d'olive, l'huile de sésame, l'huile de noyaux d'abricot, l'huile de pépin de raisin, l'huile d'amande douce, l'huile de carthame, l'huile de noisette, l'huile d'argan, le beurre de coprah ou coco, le beurre de cacao, le beurre de karité et leurs mélanges. Mieux le corps gras naturel est choisi parmi des huiles liquides à température ambiante telles que les huiles de colza, de tournesol, de soja, d'olive, de sésame, de noyaux d'abricot, de pépin de raisin, d'amande douce, de carthame, de noisette, d'argan, de coco, et leurs mélanges.

La quantité de corps gras naturel(s) utilisée dans l'étape d) va de préférence de 50 à 80 % en poids, de préférence de 60 à 75 % en poids par rapport au poids total du mélange.

Dans le mode de réalisation où les parties de l'arbre fournissant le liège sont un mélange de ses feuilles et ses fleurs, le mélange dans d) peut être réalisé à partir de :
20 à 40 % en poids, de préférence de 25 à 35 % en poids de phase grasse de l'extrait de liège, éventuellement traitée,
0,01 à 15 % en poids, de préférence de 0,1 à 10 % en poids de feuilles en poudre,
0,01 à 15 % en poids, de préférence de 0,1 à 10 % en poids de fleurs en poudre, et
50 à 80 % en poids, de préférence de 60 à 75 % en poids d'un ou plusieurs corps gras naturels,
par rapport au poids total du mélange.

De préférence, le rapport pondéral feuilles / fleurs peut varier de 1/10 à 10/1, mieux de 1/5 à 5/1.

La température utilisée dans l'étape d) est supérieure aux points de fusion de la phase grasse et du corps gras naturel ou du mélange de corps gras naturels utilisés. Le mélange de toutes les matières premières est réalisé plus particulièrement à une température allant de 30 à 80 °C, de préférence de 40 à 60 °C.

La durée de l'étape d) peut aller de 12 à 48 heures, de préférence de 24 à 48 heures et plus préférentiellement de 36 à 48 heures.

Le chauffage à l'étape e) se fait pendant une durée très courte inférieure ou égale à 20 minutes, de préférence inférieure ou égale à 15 minutes, de préférence de 1 à 10 minutes, et plus préférentiellement de 1 à 9 minutes. Cette durée correspond au temps de maintien de la température de traitement une fois cette température atteinte. Le temps de montée en température est également très court, notamment inférieur ou égal à 5 minutes, de préférence de 1 à 5 min, et plus préférentiellement de 1 à 3 minutes.

La température de chauffage de l'étape e) va de 100 à 140 °C, de préférence de 110 à 130 °C.

Tout système de chauffage thermique rapide peut être utilisé, tel que l'utilisation d'une source micro-ondes. Cette source micro-ondes utilisée dans un réacteur fermé peut permettre d'atteindre en un temps court les températures souhaitées et ainsi limiter des phénomènes d'oxydation secondaire.

Dans un mode de réalisation préféré, on utilise un générateur de micro-ondes à forte puissance utile, de préférence de l'ordre de 500 Watts à 9 000 Watts, mieux de 700 à 3000 Watts par kilogramme de mélange.

On entend par microdispersion de la phase grasse de l'extrait de liège et d'une ou des parties de l'arbre fournissant le liège réduite(s) en poudre dans le corps gras naturel, l'obtention d'une dispersion des molécules extraites dans le corps gras naturel, présentant une taille allant notamment de 0,1 à 10 µm. La taille est telle qu'il n'y a pas décantation au cours du temps.

Selon un mode de réalisation particulier de l'invention, l'étape f) est réalisée sous ondes ultrasonores. Elle est de préférence menée dans un réacteur fermé équipé d'un générateur d'ultrasons à basse fréquence de cavitation, notamment inférieure à 30 kHz et de préférence de l'ordre de 20 à 25 kHz.

La durée du traitement sous ultrasons est notamment inférieure à 30 minutes, préférentiellement elle va de 2 à 30 min, mieux encore de 10 à 20 min.

L'étape f) peut être conduite à une température allant de 30 à 80 °C, de préférence de 40 à 60 °C.

Les étapes e) et f) peuvent répétées plusieurs fois, en particulier au moins deux fois successivement, de préférence 2 ou 3 fois.

Une étape supplémentaire de macération peut être incorporée entre les deux étapes e) et f). Cette étape de macération durera de préférence au moins une heure, et mieux de 2 à 4 heures, et s'effectuera de préférence à une température allant de 20 à 100 °C, mieux de 40 à 80 °C, et encore mieux de 40 à 70 °C.

L'étape de macération peut être réalisée de toute manière connue en soi, notamment avec ou sans agitation mécanique à basse vitesse, par exemple moins de 100 tours / min, et à une température supérieure au point de fusion de la phase grasse et du corps gras naturel ou du mélange de corps gras naturels utilisés.

Cette étape de macération est avantageusement conduite en atmosphère dépourvue ou essentiellement dépourvue d'oxygène, comme les étapes d) e) et f), de préférence sous une atmosphère saturée d'azote. Elle est avantageusement conduite en l'absence de lumière ou de tout rayonnement oxydant tels que les UV, de même que peuvent l'être avantageusement les étapes d), e) et f) en l'absence de lumière ou de tout rayonnement oxydant tels que les UV pour limiter les risques de photo-oxydation et de dégradation des molécules photosensibles.

Dans un mode de réalisation particulier, après avoir effectué au moins deux fois les étapes e) et f), une étape de macération étant insérée entre chaque étape e) et chaque étape f), l'étape d) peut ensuite être à nouveau effectuée.

Une fois le procédé terminé, le mélange peut encore être filtré ou essoré à une température supérieure au point de fusion du mélange, allant généralement de 40 à 60°C pour avoir un mélange liquide et homogène au moment de la filtration.

Le produit cosmétique et/ou dermatologique obtenu selon le procédé se présente sous une forme semi-solide à température ambiante (20-25 °C), comme par exemple un beurre gras, fondant et homogène. Il fond à une température de l'ordre de 30 °C.

Le procédé de l'invention permet d'obtenir un produit présentant un faible taux d'oxydation des molécules actives extraites des matières premières, à savoir du liège, d'au moins une des parties de l'arbre fournissant le liège, et du ou des corps gras naturels.

Le produit présente une excellente stabilité à l'oxydation au cours de son stockage et de son utilisation.

La présente invention concerne aussi une composition cosmétique et/ou dermatologique comprenant au moins un produit cosmétique et/ou dermatologique selon l'invention, dans un milieu cosmétiquement ou dermatologiquement acceptable.

La quantité de produit(s) cosmétique(s) et/ou dermatologique(s) selon l'invention va de préférence de 0,1 à 10 % en poids, mieux de 0,5 à 8 % en poids, et préférentiellement de 1 à 5 % en poids par rapport au poids total de la composition.

Le milieu cosmétiquement ou dermatologiquement acceptable comprend soit une seule phase grasse (huile de soin, sérum huileux) soit une émulsion comprenant une phase grasse et une phase aqueuse (lait, crème, microémulsion). La phase grasse peut contenir une ou plusieurs huiles végétales choisies parmi les corps gras naturels définis ci-dessus.

La composition cosmétique et/ou dermatologique peut comprendre d'autres ingrédients cosmétiques et/ou dermatologiques. Ils peuvent être choisis parmi des agents tensioactifs anioniques, cationiques, amphotères ou non-ioniques, plus particulièrement non-ioniques comme, par exemple, les alkyl glycosides, et plus particulièrement les cetearyl glucosides ; des épaississants ou gélifiants comme les gommes de polysaccharides, par exemple, les gommes de cellulose ou les gommes de xanthane, et les copolymères de vinylypyrrolidone tels que le copolymère vinylpyrrolidone/acryldiméthyltaurate d'ammonium ; des pigments comme le dioxyde de titane ; des conservateurs ; des agents régulateurs du pH ; des argiles comme l'hectorite ou la bentonite ; et leurs mélanges.

La composition cosmétique et/ou dermatologique peut se trouver sous la forme de crème, lait, émulsion ou sérum huileux.

L'invention est relative à un procédé de traitement cosmétique des signes du vieillissement de la peau consistant à appliquer sur la peau, la composition telle que décrite ci-dessus.

Le produit cosmétique et/ou dermatologique tel que décrit ci-dessus, peut être utilisé en cosmétique pour hydrater les couches supérieures de la peau.

L'invention concerne aussi le produit cosmétique et/ou dermatologique pour son utilisation comme médicament.

En particulier, il est utilisé dans le traitement des érythèmes des peaux sensibles ou réactives.

Par peaux sensibles ou réactives, on entend des peaux qui se caractérisent par la présence de rougeurs locales ou diffuses ainsi que par des sensations de chaleur, de démangeaison ou de picotements. Elles se déshydratent facilement, créant des sensations de tiraillement.

En outre, l'invention porte sur l'utilisation du produit selon l'invention dans la protection de la peau vis-à-vis des rayons ultraviolets ainsi que dans le traitement des érythèmes solaires.

L'invention est illustrée par les exemples suivants.

### Exemples

### Exemple 1

Après avoir traité du liège avec du CO₂ supercritique à une température de 60 °C et sous une pression de 10-10,5 MPa (100-105 bars), en présence d'eau en une quantité de 0,02 % en poids par rapport au poids du CO₂, on récupère un extrait de liège brut.

On sépare ensuite la phase grasse de la phase liquide par décantation statique à température ambiante (20-25 °C).

La phase grasse est ensuite introduite dans un réacteur inox à double enveloppe, puis chauffée 53°C sous agitation pendant 15 minutes.

Auparavant, on a récolté des fleurs et des feuilles de chêne-liège au moment de la floraison, et on les a laissées sécher à 30°C pendant 1 semaine. On les a ensuite réduites en poudre par pulvérisation dans un malaxeur à hélices à une température de -80°C. On a ainsi obtenu une poudre fine et régulière de granulométrie de 200 à 400 µm.

La phase grasse traitée et les poudres ont été mélangées dans les proportions suivantes avec de l'huile de coprah :
- 25 % en poids de phase grasse traitée,
- 3 % en poids de feuilles en poudre,
- 7 % en poids de fleurs en poudre et
- 65 % en poids d'huile de coprah.

On a introduit ce mélange dans un réacteur inox fermé, équipé d'un agitateur. L'étape d) est ensuite conduite à une température de 40°C pendant une durée de 48 h, suivie de l'étape e) à 125 °C, pendant une durée de 9 minutes, avec un générateur micro-ondes à forte puissance utile, de l'ordre de 1 KW par kilogramme de mélange.

On a laissé macérer le mélange de l'étape e) pendant 2 heures à 60 °C.

Ensuite, l'étape f) a été réalisée par cavitation sous ondes ultrasonores dans un réacteur fermé équipé d'un générateur d'ultrasons à basse fréquence de cavitation de 25 kHz pendant 10 min.

Les étapes e), de macération et f) ont été réalisées deux fois successivement. L'étape d) est réalisée à nouveau avant l'essorage final.

Une atmosphère saturée d'azote a été maintenue pendant la réalisation de toutes les étapes d) à f).

On a obtenu un produit sous la forme d'un beurre gras à température ambiante, fondant, brillant, homogène, et de couleur gris beige.

### Exemple 2

On a répété le procédé de l'exemple 1 avec le mélange suivant :
- 30 % en poids de phase grasse traitée,
- 5 % en poids de feuilles en poudre,
- 5 % en poids de fleurs en poudre et
- 60 % en poids d'huile de coprah.

### Exemple 3

On a répété le procédé de l'exemple 1 avec le mélange suivant :
- 25 % en poids de phase grasse traitée,
- 4 % en poids de feuilles en poudre,
- 1 % en poids de fleurs en poudre et
- 70 % en poids d'huile de coprah.

### Exemple 4

On a préparé des compositions cosmétiques et/dermatologiques A, B et C selon l'invention, dans un milieu constitué d'huile d'olive, comme indiqué ci-dessous. Les proportions sont exprimées en % en poids par rapport au poids total de la composition.

| Composition | A | B | C |
|---|---|---|---|
| Produit obtenu à l'exemple 1, 2 ou 3 | 1 % | 2 % | 3 % |
| Huile d'olive | 99 % | 98 % | 97 % |

### Exemple 5

On a préparé une composition cosmétique et/dermatologique selon l'invention, sous la forme d'un gel, à partir des ingrédients suivants, les proportions étant exprimées en % en poids par rapport au poids total de la composition :

| | | |
|---|---|---|
| Produit obtenu à l'exemple 1, 2 ou 3 | | 5 % |
| Huile d'olive raffinée CODEX | | 5 % |
| Copolymère vinylpyrrolidone/acryldiméthyltaurate d'ammonium vendu sous la dénomination commerciale Aristoflex par la société Clariant | | 2 % |
| Conservateur | | 0,1 % |
| Eau | qsp | 100 % |

### Exemple 6

On a préparé une crème de base à partir des ingrédients suivants, les proportions étant exprimées en % en poids par rapport au poids total de la crème de base :

**Phase huileuse**

| | |
|---|---|
| Huile désodorisée de tournesol oléique | 20 % |
| Alcool cétéarylique/cétéaryl wheat straw glycoside (rapport pondéral 25/75) vendu sous la dénomination commerciale xyliance par la société Soliance | 2,5 % |
| Tensioactif vendu sous la dénomination commercial Montanov 68 par la société Seppic | 2,5 % |

**Phase aqueuse 1**

| | | |
|---|---|---|
| Gomme de xanthane/gomme de cellulose/hectorite (rapport pondéral 33/34/33) vendu sous la dénomination commerciale Thickagent par la société Sensiant | | 20 % |
| Acide lactique | | 0,1 % |
| Conservateur | | 0,1 % |
| Eau | qsp | 100 % |

**Phase aqueuse 2**

| | |
|---|---|
| Glycérine | 4 % |
| Dioxyde de titane | 0,25 % |

On a préparé la phase aqueuse 1 sans les conservateurs et l'acide lactique, et la phase aqueuse 2 séparément puis on a ajouté la phase aqueuse 2 à la phase aqueuse 1 ainsi préparée, sous agitation à une température de 70 °C.

Après avoir mélangé les ingrédients de la phase huileuse, on l'a chauffée jusqu'à la faire fondre, puis on y a incorporé la phase aqueuse préparée ci-dessus, toujours sous agitation à température de 70°C.

Après l'incorporation de la phase aqueuse dans la phase huileuse, on a laissé refroidir dans un bain-marie d'eau froide, tout en continuant l'agitation. A la température de 30 °C, on a ajouté les conservateurs et l'acide lactique.

On ensuite laissé refroidir la crème ainsi obtenue et on a mélangé 95 % en poids de cette crème de base et 5 % en poids de produit de l'exemple 3, par rapport au poids total de la composition finale.

### Exemple 7 : Test ORAC

La capacité à piéger les radicaux libres, signe d'une activité anti-âge, peut être mesurée par le test in-vitro ORAC. Ce test permet d'évaluer la capacité d'un extrait végétal à bloquer les réactions radicalaires oxydatives. Il mesure les dommages causés par des radicaux libres sur un marqueur fluorescent, ce qui se traduit par une baisse de l'intensité de la fluorescence mesurée en spectrophotométrie à fluorescence. La présence d'antioxydants dans un produit à tester empêche les radicaux libres d'agir sur le composé fluorescent.

On utilise le dichlorhydrate de 2,2'-azobis-2-amidinopropane (AAPH) comme générateur d'espèces réactives à l'oxygène (ROS), et la fluorescéine comme marqueur fluorescent. Le Trolox® (acide 6-hydroxy-2,5,7,8-tétraméthylchroman-2-carboxylique), analogue de la vitamine E, est utilisé comme référence externe et les résultats ORAC sont exprimés en Trolox® équivalent (TE) par unité de poids d'échantillon.

Le test a été réalisé sur le produit préparé à l'exemple 3 et sur l'huile de coprah seule, ainsi que sur les extraits suivants :
- extrait comparatif 1 obtenu avec le procédé selon l'invention, notamment celui décrit à l'exemple 1, sans utiliser de fleurs et de feuilles, par mélange à l'étape d) de 25 % en poids de phase grasse traitée et de 75 % d'huile de coprah,
- extrait comparatif 2 obtenu avec les étapes d) à f) du procédé selon l'invention, notamment celui décrit à l'exemple 1, sans utiliser de phase grasse traitée, par mélange à l'étape d) de 4% en poids de fleurs en poudre, de 1 % en poids de feuilles en poudre et de 95 % en poids d'huile de coprah.

Les résultats sont regroupés dans le tableau ci-dessous.

| Nature de l'échantillon | Valeur ORAC en micromoles Trolox équivalent par kg de produit |
|---|---|
| Huile de coprah seule | 210 |
| Extrait comparatif 1 | 8 290 |
| Extrait comparatif 2 | 4 995 |
| Produit de l'exemple 3 | 17 000 |

Le test ORAC montre un effet synergique obtenu avec le produit selon l'invention, comparé avec l'effet obtenu avec la phase grasse traitée seule et le mélange de poudres de feuilles et de fleurs. On peut noter une augmentation de 28 %.

### Exemple 8 : viabilité cellulaire

L'Alamar Blue est une sonde fluorogène bleue qui pénètre passivement dans les cellules où elle est réduite par les enzymes d'oxydoréduction intracellulaires. La forme bleue non fluorescente (résazurine) devient alors rouge et fluorescente (résorufine). En effet, lorsque les cellules sont en vie, elles maintiennent un environnement réducteur dans leurs compartiments intracellulaires. La résazurine est ainsi réduite en résorufine qui diffuse dans le milieu de culture où elle peut être dosée.

Une solution mère à 0,1mg/mL d'Alamar blue est préparée dans du PBS, solution physiologique tampon - Phosphate Buffered Saline -, puis diluée au 11^{ième} dans du milieu de culture à 2,5%. La solution fille est distribuée à 200µL/puits d'une microplaque. Chaque composition testée comprenant 1 %, 2 % ou 3% en poids de produit de l'exemple 1 dans de l'huile d'olive est déposée dans 6 puits d'une même colonne et chaque test est répété au minimum 3 fois. La microplaque est alors placée dans l'incubateur pendant 6 heures puis lue par le cytofluorimètre Safire-TECAN (λexc=535nm, λem=600nm).

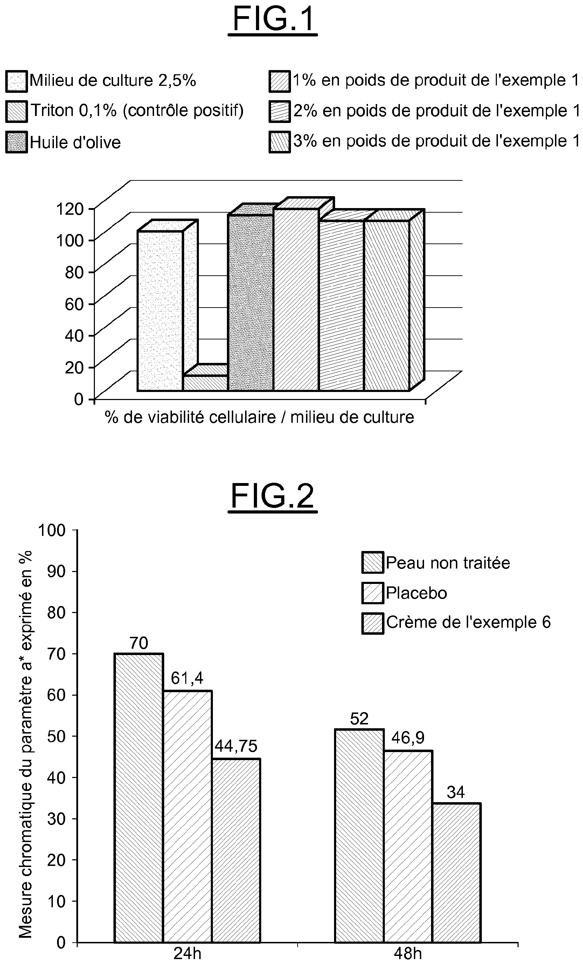

Au vu des résultats indiqués dans la Fig. 1, la viabilité cellulaire n'est pas altérée quelle que soit la concentration de produit testée.

### Exemple 9 montrant l'action préventive sur un érythème

Un test clinique a été réalisée sur 9 femmes, d'origine caucasienne, de 20 à 52 ans, présentant un phototype II ou III.

Pour chaque volontaire, la Dose Erythémale Minimum individuelle (D.E.M.i) a été déterminée au niveau de l'avant-bras. Il s'agit de la dose d'UV minimale capable d'induire un érythème (rougeur) chez le volontaire. La D.E.M.i varie d'un individu à l'autre.

### Modalités d'application des produits

4 zones « d'application » de 16 cm² chacune ont été déterminées au niveau des avant-bras :
▪ Zone 1 : Application de 3 mg/cm² de la crème de l'exemple 6
▪ Zone 2 : Application de 3 mg/cm² du placebo (crème de base décrite à l'exemple 6)
▪ Zone 3 : Zone non traitée et exposée aux U.V.A & B
▪ Zone 4 : Zone non traitée et non exposée

La crème a été appliquée 2 fois par jour (matin et soir) pendant une durée de 11 jours.

### Exposition UV

Le douzième jour, on a exposé chaque volontaire à une dose équivalente à 1,5 fois la D.E.M.i à l'aide d'un simulateur solaire Oriel 1600 Watts émettant des rayonnements U.V.A & U.V.B.

### Evaluation :

On a évalué l'érythème solaire à l'aide de mesures colorimétriques en utilisant l'appareil Chromamèter CR400™, et on a mesuré le paramètre a* * (axe vert-rouge) de la notation 1976 CIE La*b* qui définit un espace colorimétrique dans lequel chaque couleur est définie par trois paramètres L*, a* et b*.

Le paramètre a* a été mesuré 30 minutes, 2 heures puis 24 heures après l'exposition. Les résultats moyens sont montrés dans la Fig. 2.

On a remarqué l'apparition d'un érythème moins important avec les zones traitées avec la crème selon l'invention.

### Exemple 10 montrant l'action apaisante sur un érythème

Un test clinique a été réalisée sur 10 femmes, d'origine caucasienne, de 20 à 56 ans, présentant un phototype II ou III.

Pour chaque volontaire, la Dose Erythémale Minimum individuelle (D.E.M.i) a été déterminée au niveau de l'avant-bras. Il s'agit de la dose d'UV minimale capable d'induire un érythème (rougeur) chez le volontaire. La D.E.M.i varie d'un individu à l'autre.

### Modalités d'application des produits

4 zones « d'application » de 16 cm² chacune ont été déterminées au niveau des avant-bras :
▪ Zone 1 : Application de 3 mg/cm² de la crème de l'exemple 6
▪ Zone 2 : Application de 3 mg/cm² du placebo (crème de base décrite à l'exemple 6)
▪ Zone 3 : Zone non traitée et exposée aux U.V.A & B
▪ Zone 4 : Zone non traitée et non exposée

La crème a été appliquée 3 fois à 30 min, 2 h et 24 h après l'exposition aux UV qui a consisté à exposer chaque volontaire à une dose équivalente à 1,5 fois la D.E.M.i à l'aide d'un simulateur solaire Oriel 1600 Watts émettant des rayonnements U.V.A & U.V.B.

### Evaluation :

On a évalué l'érythème solaire à l'aide de mesures colorimétriques en utilisant l'appareil Chromamèter CR400™, et on a mesuré le paramètre a* * (axe vert-rouge) de la notation 1976 CIE La*b* qui définit un espace colorimétrique dans lequel chaque couleur est définie par trois paramètres L*, a* et b*.

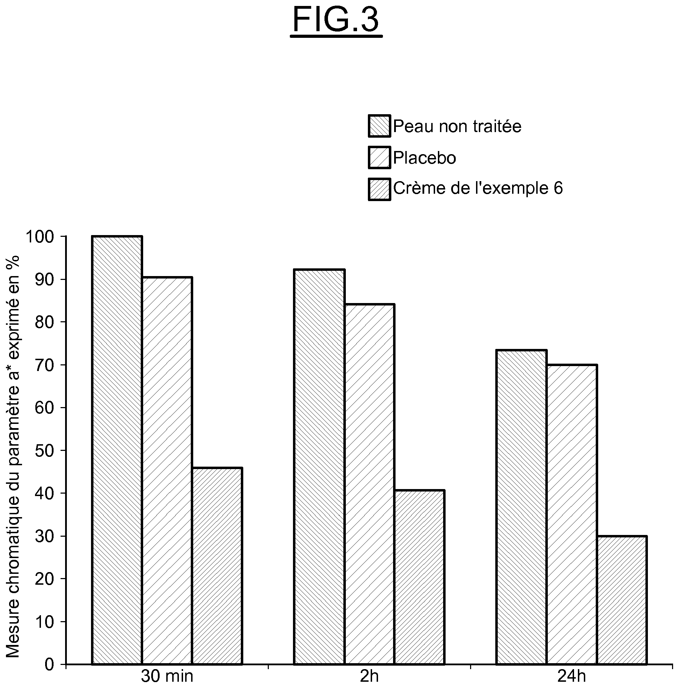

Le paramètre a* a été mesuré 30 minutes, 2 heures puis 24 heures et 48 heures après l'exposition. Les résultats moyens sont montrés dans la Fig. 3.

On a remarqué l'apparition d'un érythème moins important avec les zones traitées avec la crème selon l'invention.

## Revendications

1. Produit cosmétique et/ou dermatologique comprenant :
0,5 à 10 % en poids, de préférence de 1 à 7 % en poids d'acides gras en C₆₋₃₀, de préférence en C₆₋₂₄, saturés ou insaturés, linéaires ou ramifiés,
3 à 15 % en poids, de préférence de 5 à 10 % en poids d'alcools gras en C₆₋₃₀, saturés ou insaturés, linéaires ou ramifiés,
0,5 à 10 % en poids, de préférence de 1 à 5 % en poids d'un ou plusieurs phytostérols,
0,1 à 10 % en poids, de préférence de 0,5 à 5 % en poids d'un ou plusieurs terpènes différents du squalène,
1 à 15 % en poids, de préférence de 3 à 10 % en poids de squalène, et
50 à 90 % en poids, de préférence de 55 à 85 % en poids de triglycérides,
par rapport au poids total du produit.

2. Produit cosmétique et/ou dermatologique selon la revendication 1, **caractérisé en ce que** les acides gras en C₆₋₃₀ sont linéaires et saturés, mono-insaturés ou di-insaturés.

3. Produit cosmétique et/ou dermatologique selon la revendication 1 ou 2, **caractérisé en ce que** les acides gras en C₆₋₃₀ sont choisis parmi l'acide hexanoïque (ou caproïque), l'acide octanoïque (ou caprylique), l'acide décanoïque (ou caprique), l'acide laurique, l'acide myristique, l'acide palmitique, l'acide margarique, l'acide stéarique, l'acide arachidique, l'acide béhénique, l'acide lignocérique, l'acide palmitoléique, l'acide oléique, l'acide linoléique, et leurs mélanges.

4. Produit cosmétique et/ou dermatologique selon l'une quelconque des revendications précédentes, **caractérisé en ce que** les acides gras en C₆₋₃₀ sont sous la forme d'un mélange de
0,2 à 1,5 % en poids d'acide hexanoïque (ou caproïque),
4 à 20 % en poids d'acide octanoïque (ou caprylique),
2 à 15 % en poids d'acide décanoïque (ou caprique),
30 à 60 % en poids d'acide laurique,
10 à 30 % en poids d'acide myristique,
4 à 12 % en poids d'acide palmitique,
1 à 6 % en poids d'acide stéarique,
1 à 11 % en poids d'acide oléique,
0,5 à 4 % en poids d'acide linoléique et
moins de 3 % en poids d'acide gras en C₂₀₋₃₀.

5. Produit cosmétique et/ou dermatologique selon l'une quelconque des revendications précédentes, **caractérisé en ce que** les terpènes différent du squalène sont la friedéline, la bétuline, le lupen-3-one, la sitost-4-én-3-one, et des alcools triterpéniques comme le lupéol, le bétulinol, l'α-amyrine et l'érythrodiol.

6. Produit cosmétique et/ou dermatologique selon l'une quelconque des revendications précédentes, **caractérisé en ce qu'**il contient plus de 20 % en poids, mieux de 20 à 50 % en poids, de composés terpéniques incluant le squalène, les terpènes différents du squalène et les phytostérols.

7. Produit cosmétique et/ou dermatologique selon l'une quelconque des revendications précédentes, **caractérisé en ce que** les triglycérides sont des esters de glycérol et d'acides gras en C₄₋₃₀, de préférence en C₄₋₂₄.

8. Procédé de préparation d'un produit cosmétique et/ou dermatologique selon l'une quelconque des revendications 1 à 7, à partir de liège et d'au moins une partie de l'arbre fournissant le liège choisie parmi ses feuilles, ses fleurs, ses racines, ses bourgeons et leurs mélanges, de préférence parmi ses feuilles, ses fleurs et leurs mélanges, comprenant les étapes qui consistent à :
a) mettre le liège en contact avec du CO₂ supercritique, à une température allant de 10 à 120 °C et sous une pression allant de 1 à 60 MPa (10 à 600 bars), éventuellement en présence d'un co-solvant,
b) récupérer l'extrait de liège entrainé par le CO₂ supercritique, sous la forme d'un mélange de phases aqueuse et grasse,
c) séparer la phase grasse de l'extrait de liège de la phase aqueuse,
d) mélanger ladite phase grasse de l'extrait de liège avec une ou des parties de l'arbre fournissant le liège réduite(s) en poudre, et un ou plusieurs corps gras naturels, à une température supérieure aux températures de fusion de la phase grasse et du ou des corps gras naturels, sous une atmosphère dépourvue ou essentiellement dépourvue d'O₂,
e) chauffer le mélange obtenu à une température allant de 100 à 140 °C, de préférence de 110 à 130 °C, pendant une durée inférieure à 20 minutes, de préférence inférieure à 15 minutes, sous une atmosphère dépourvue ou essentiellement dépourvue d'O₂,
f) effectuer une microdispersion de ladite phase grasse de l'extrait de liège, d'une ou des parties de l'arbre fournissant le liège réduite(s) en poudre dans le ou les corps gras naturels, à une température supérieure aux températures de fusion de la phase grasse et du ou des corps gras naturels, sous une atmosphère dépourvue ou essentiellement dépourvue d'O₂, et à
g) éventuellement répéter les deux étapes e) et f).

9. Procédé selon la revendication 8, **caractérisé en ce que** le co-solvant de l'étape a) est choisi parmi l'eau et les mélanges d'eau et d'alcool en C₁₋₄.

10. Procédé selon la revendication 8 ou 9, **caractérisé en ce que** le co-solvant de l'étape a) est présent en une quantité allant de 0,01 à 10 % en poids, de préférence de 0,02 à 1 % en poids par rapport au poids du CO₂ supercritique utilisé.

11. Procédé selon l'une quelconque des revendications 8 à 10, **caractérisé en ce que** la phase grasse de l'extrait de liège obtenue à l'étape c) est traitée avant l'étape d), par chauffage sous agitation à une température supérieure au point de fusion de la phase grasse, sous une atmosphère dépourvue ou essentiellement dépourvue d'O₂, de préférence pendant 15 à 60 minutes.

12. Procédé selon la revendication 11, **caractérisé en ce que** la température de chauffage va de 40 à 90 °C, de préférence de 50 à 60 °C.

13. Procédé selon l'une quelconque des revendications 8 à 12, **caractérisé en ce que** la ou les parties de l'arbre fournissant le liège est ou sont dans un premier temps, récoltée(s), déshydratée(s) à l'air, séchée(s) à une température inférieure ou égale à 40 °C pour obtenir une teneur en eau résiduelle allant de 5 à 15 % en poids, de préférence de 8 à 10 % en poids par rapport au poids total du mélange obtenu, puis dans un deuxième temps, broyée(s) et pulvérisée(s) à une température allant de -18 à -80 °C, avant leur utilisation dans l'étape d).

14. Procédé selon l'une quelconque des revendications 8 à 13, **caractérisé en ce que** la ou les parties de l'arbre fournissant le liège réduite(s) en poudre présentent une granulométrie allant de 100 µm à 1 mm.

15. Procédé selon l'une quelconque des revendications 8 à 14, **caractérisé en ce que** le ou les corps gras naturels sont choisis parmi l'huile de colza, l'huile de tournesol, l'huile de soja, l'huile d'olive, l'huile de sésame, l'huile de noyaux d'abricot, l'huile de pépin de raisin, l'huile d'amande douce, l'huile de carthame, l'huile de noisette, l'huile d'argan, le beurre de coprah ou coco, le beurre de cacao, le beurre de karité et leurs mélanges.

16. Procédé selon l'une quelconque des revendications 8 à 15, **caractérisé en ce que** les parties de l'arbre fournissant le liège sont ses feuilles et ses fleurs et le mélange dans d) est réalisé à partir de :
20 à 40 % en poids de phase grasse de l'extrait de liège,
0,01 à 15 % en poids de feuilles en poudre,
0,01 à 15 % en poids de fleurs en poudre, et
50 à 80 % d'un ou plusieurs corps gras naturels,
par rapport au poids total du mélange.

17. Procédé selon l'une quelconque des revendications 8 à 16, **caractérisé en ce qu'**entre chaque étape e) et f) est réalisée une étape de macération pendant plus d'une heure, de préférence de 2 à 4 heures.

18. Procédé selon l'une quelconque des revendications 8 à 17, **caractérisé en ce que** l'étape f) est réalisée pendant une durée inférieure à 30 minutes, de préférence allant de 2 à 30 minutes.

19. Produit cosmétique et/ou dermatologique **caractérisé par le fait qu'**il est susceptible d'être obtenu par le procédé selon l'une quelconque des revendications 8 à 18.

20. Produit cosmétique et/ou dermatologique selon l'une quelconque des revendications 1 à 7, **caractérisé par le fait qu'**il est susceptible d'être obtenu par le procédé selon l'une quelconque des revendications 8 à 18.

21. Produit cosmétique et/ ou dermatologique selon la revendication 19 ou 20, se présentant sous forme semi-solide à une température de 20-25°C et ayant un point de fusion de l'ordre de 30°C.

22. Composition cosmétique et/ou dermatologique comprenant le produit cosmétique et/ou dermatologique selon l'une quelconque des revendications 1 à 7 et 19 à 21 dans un milieu cosmétiquement et/ou dermatologiquement acceptable.

23. Composition cosmétique et/ou dermatologique selon la revendication 22, **caractérisée en ce que** le produit cosmétique et/ou dermatologique est présent en une quantité allant de 0,1 à 10 % en poids, mieux de 0,5 à 8 % en poids, et préférentiellement de 1 à 5 % en poids par rapport au poids total de la composition.

24. Composition cosmétique et/ou dermatologique selon la revendication 22 ou 23, **caractérisée en ce que** le milieu cosmétiquement et/ou dermatologiquement acceptable comprend une seule phase grasse ou une émulsion comprenant une phase grasse et une phase aqueuse.

25. Procédé de traitement cosmétique des signes du vieillissement de la peau, **caractérisé en ce qu'**il consiste à appliquer sur la peau, une composition selon l'une quelconque des revendications 22 à 24.

26. Utilisation du produit selon l'une quelconque des revendications 1 à 7 et 19 à 21, en cosmétique, pour hydrater les couches supérieures de la peau.

27. Produit selon l'une quelconque des revendications 1 à 7 et 19 à 21, pour son utilisation comme médicament.

28. Produit selon l'une quelconque des revendications 1 à 7 et 19 à 21, pour son utilisation dans le traitement des érythèmes des peaux sensibles ou réactives.

29. Produit selon l'une quelconque des revendications 1 à 7 et 19 à 21, pour son utilisation dans la protection de la peau vis-à-vis des rayons ultraviolets.

30. Produit selon l'une quelconque des revendications 1 à 7 et 19 à 21, pour son utilisation dans le traitement des érythèmes solaires.

## Patentansprüche

1. Kosmetikprodukt und/oder dermatologisches Produkt, das Folgendes umfasst:
0,5 bis 10 Ges.-%, vorzugsweise 1 bis 7 Ges.-%, gesättigte oder ungesättigte, geradkettige oder verzweigte C₆₋₃₀-Fettsäuren, vorzugsweise C₆₋₂₄-Fettsäuren,
3 bis 15 Gew.-%, vorzugsweise 5 bis 10 Gew.-%, gesättigte oder ungesättigte, geradkettige oder verzweigte C₆₋₃₀-Fettalkohole,
0,5 bis 10 Gew.-%, vorzugsweise 1 bis 5 Gew.-%, an einem oder mehreren Phytosterolen,
0,1 bis 10 Gew.-%, vorzugsweise 0,5 bis 5 Gew.-%, an einem oder mehreren Terpenen, die unterschiedlich zu Squalen sind,
1 bis 15 Gew.-%, vorzugsweise 3 bis 10 Gew.-%, Squalen sowie
50 bis 90 Gew.-%, vorzugsweise 55 bis 85 Gew.-%, Triglyceride
in Bezug auf das Gesamtgewicht des Produkts.

2. Kosmetikprodukt und/oder dermatologisches Produkt nach Anspruch 1, **dadurch gekennzeichnet, dass** die C₆₋₃₀-Fettsäuren geradkettig und gesättigt, einfach ungesättigt oder zweifach ungesättigt sind.

3. Kosmetikprodukt und/oder dermatologisches Produkt nach Anspruch 1 oder 2, **dadurch gekennzeichnet, dass** die C₆₋₃₀-Fettsäuren aus Hexansäure (oder Capronsäure), Octansäure (oder Caprylsäure), Decansäure (oder Caprinsäure), Laurinsäure, Myristinsäure, Palmitinsäure, Margarinsäure, Stearinsäure, Arachinsäure, Behensäure, Lignocerinsäure, Palmitoleinsäure, Ölsäure, Linolsäure und ihren Mischungen ausgewählt ist.

4. Kosmetikprodukt und/oder dermatologisches Produkt nach einem der vorhergehenden Ansprüche, **dadurch gekennzeichnet, dass** die C₆₋₃₀-Fettsäuren in Form einer Mischung aus
0,2 bis 1,5 Gew.-% Hexansäure (oder Capronsäure),
4 bis 20 Gew.-% Octansäure (oder Caprylsäure),
2 bis 15 Gew.-% Decansäure (oder Caprinsäure),
30 bis 60 Gew.-% Laurinsäure,
10 bis 30 Gew.-% Myristinsäure,
4 bis 12 Gew.-% Palmitinsäure,
1 bis 6 Gew.-% Stearinsäure,
1 bis 11 Gew.-% Ölsäure,
0,5 bis 4 Gew.-% Linolsäure und
weniger als 3 Ges.-% C₂₀₋₃₀-Fettsäure
vorliegen.

5. Kosmetikprodukt und/oder dermatologisches Produkt nach einem der vorhergehenden Ansprüche, **dadurch gekennzeichnet, dass** es sich bei den von Squalen unterschiedlichen Terpenen um Friedelin, Betulin, Lupen-3-on, Sitost-4-en-3-on und Triterpenalkohole wie Lupeol, Betulinol, α-Amyrin und Erythrodiol handelt.

6. Kosmetikprodukt und/oder dermatologisches Produkt nach einem der vorhergehenden Ansprüche, **dadurch gekennzeichnet, dass** es mehr als 20 Gew.-%, besser 20 bis 50 Gew.-%, Terpenverbindungen einschließlich Squalen, von Squalen unterschiedliche Terpene und Phytosterole enthält.

7. Kosmetikprodukt und/oder dermatologisches Produkt nach einem der vorhergehenden Ansprüche, **dadurch gekennzeichnet, dass** es sich bei den Triglyceriden um Ester von Glycerin und C₄₋₃₀-Fettsäuren, vorzugsweise C₄-₂₄-Fettsäuren, handelt.

8. Verfahren zur Herstellung eines Kosmetikprodukts und/oder dermatologischen Produkts nach einem der Ansprüche 1 bis 7 ausgehend von Kork und mindestens einem Teil des Baums, der den Kork produziert, ausgewählt aus seinen Blättern, seinen Blüten, seinen Wurzeln, seinen Knospen und ihren Mischungen, vorzugsweise aus seinen Blättern, seinen Blüten und ihren Mischungen, umfassend die Schritte, die aus Folgendem bestehen:
a) Inkontaktbringen des Korks mit überkritischem CO₂ bei einer Temperatur von 10 bis 120°C und einem Druck von 1 bis 60 MPa (10 bis 600 bar), gegebenenfalls in Gegenwart eines Hilfslösungsmittels,
b) Gewinnen des von dem überkritischen CO₂ mitgeführten Korkextrakts in Form einer Mischung von wässrigen und fetten Phasen,
c) Trennen der Fettphase des Korkextrakts von der wässrigen Phase,
d) Mischen der Fettphase des Korkextrakts mit einem oder mehreren Teilen des Baums, der den Kork produziert, in Pulverform und einem oder mehreren natürlichen Fettkörpern bei einer Temperatur oberhalb der Schmelzpunkte der Fettphase und des natürlichen Fettkörpers bzw. der natürlichen Fettkörper unter einer O₂-freien oder im Wesentlichen O₂-freien Atmosphäre,
e) Erhitzen der erhaltenen Mischung auf eine Temperatur von 100 bis 140°C, vorzugsweise 110 bis 130°C, über einen Zeitraum von weniger als 20 Minuten, vorzugsweise weniger als 15 Minuten, unter einer O₂-freien oder im Wesentlichen O₂-freien Atmosphäre,
f) Herstellen einer Mikrodispersion der Fettphase des Korkextrakts, von einem oder mehreren Teilen des Baums, der den Kork produziert, in Pulverform in dem oder den natürlichen Fettkörper(n) bei einer Temperatur oberhalb der Schmelzpunkte der Fettphase und des natürlichen Fettkörpers bzw. der natürlichen Fettkörper unter einer O₂-freien oder im Wesentlichen O₂-freien Atmosphäre, sowie
g) gegebenenfalls Wiederholen der beiden Schritte e) und f).

9. Verfahren nach Anspruch 8, **dadurch gekennzeichnet, dass** das Hilfslösungsmittel von Schritt a) aus Wasser und Mischungen von Wasser und C₁₋₄-Alkohol ausgewählt ist.

10. Verfahren nach Anspruch 8 oder 9, **dadurch gekennzeichnet, dass** das Hilfslösungsmittel von Schritt a) in einer Menge von 0,01 bis 10 Gew.-%, vorzugsweise von 0,02 bis 1 Gew.-%, in Bezug auf das Gewicht des eingesetzten überkritischen CO₂ vorliegt.

11. Verfahren nach einem der Ansprüche 8 bis 10, **dadurch gekennzeichnet, dass** die in Schritt c) erhaltene Fettphase des Korkextrakts vor Schritt d) durch vorzugsweise 15- bis 60-minütiges Erhitzen unter Rühren auf eine Temperatur oberhalb des Schmelzpunkts der Fettphase unter einer O₂-freien oder im Wesentlichen O₂-freien Atmosphäre behandelt wird.

12. Verfahren nach Anspruch 11, **dadurch gekennzeichnet, dass** die Erhitzungstemperatur 40 bis 90°C, vorzugsweise 50 bis 60°C, beträgt.

13. Verfahren nach einem der Ansprüche 8 bis 12, **dadurch gekennzeichnet, dass** der oder die Teile des Baums, der den Kork produziert, in einer ersten Stufe geerntet, luftgetrocknet, auf eine Temperatur von 40°C oder darunter getrocknet wird/werden, um zu einem Restfeuchtegehalt von 5 bis 15 Gew.-%, vorzugsweise 8 bis 10 Gew.-%, in Bezug auf das Gesamtgewicht der erhaltenen Mischung zu gelangen und in einer zweiten Stufe bei einer Temperatur von -18 bis -80°C gemahlen und pulverisiert werden, um anschließend in Schritt d) eingesetzt zu werden.

14. Verfahren nach einem der Ansprüche 8 bis 13, **dadurch gekennzeichnet, dass** der pulverförmige bzw. die pulverförmigen Teil(e) des Baums, der den Kork produziert, eine Korngrößenverteilung von 100 µm bis 1 mm aufweisen.

15. Verfahren nach einem der Ansprüche 8 bis 14, **dadurch gekennzeichnet, dass** der oder die natürliche(n) Fettkörper aus Rapsöl, Sonnenblumenöl, Sojaöl, Olivenöl, Sesamöl, Aprikosenkernöl, Traubenkernöl, Süßmandelöl, Safloröl, Haselnussöl, Arganöl, Kopra- oder Kokosnussbutter, Kakaobutter, Karitébutter und ihren Mischungen ausgewählt ist.

16. Verfahren nach einem der Ansprüche 8 bis 15, **dadurch gekennzeichnet, dass** die Teile des Baums, der den Kork produziert, seine Blätter und seine Blüten sind und die Mischung in d) mit:
20 bis 40 Gew.-% Fettphasen des Korkextrakts,
0,01 bis 15 Gew.-% pulverförmigen Blättern,
0,01 bis 15 Gew.-% pulverförmigen Blüten und
50 bis 80 Gew.-% an einem oder mehreren natürlichen Fettkörper(n)
in Bezug auf das Gesamtgewicht der Mischung hergestellt wird.

17. Verfahren nach einem der Ansprüche 8 bis 16, **dadurch gekennzeichnet, dass** zwischen jedem Schritt e) und f) ein mehr als einstündiger, vorzugsweise zweibis vierstündiger, Mazerationsschritt erfolgt.

18. Verfahren nach einem der Ansprüche 8 bis 17, **dadurch gekennzeichnet, dass** Schritt f) über einen Zeitraum von weniger als 30 Minuten, vorzugsweise von 2 bis 30 Minuten, erfolgt.

19. Kosmetikprodukt und/oder dermatologisches Produkt, **dadurch gekennzeichnet, dass** es nach dem Verfahren nach einem der Ansprüche 8 bis 18 erhältlich ist.

20. Kosmetikprodukt und/oder dermatologisches Produkt nach einem der Ansprüche 1 bis 7, **dadurch gekennzeichnet, dass** es nach dem Verfahren nach einem der Ansprüche 8 bis 18 erhältlich ist.

21. Kosmetikprodukt und/oder dermatologisches Produkt nach Anspruch 19 oder 20, das in bei einer Temperatur von 20-25°C halbfester Form vorliegt und einen Schmelzpunkt um 30°C aufweist.

22. Kosmetikzusammensetzung und/oder dermatologische Zusammensetzung, umfassend das Kosmetikprodukt und/oder dermatologische Produkt nach einem der Ansprüche 1 bis 7 und 19 bis 21 in einem kosmetisch und/oder dermatologisch unbedenklichen Medium.

23. Kosmetikzusammensetzung und/oder dermatologische Zusammensetzung nach Anspruch 22, **dadurch gekennzeichnet, dass** das Kosmetikprodukt und/oder dermatologische Produkt in einer Menge von 0,1 bis 10 Gew.-%, besser 0,5 bis 8 Gew.-%, vorzugsweise 1 bis 5 Gew.-% in Bezug auf das Gesamtgewicht der Zusammensetzung vorliegt.

24. Kosmetikzusammensetzung und/oder dermatologische Zusammensetzung nach Anspruch 22 oder 23, **dadurch gekennzeichnet, dass** das kosmetisch und/oder dermatologisch unbedenkliche Medium nur eine Fettphase oder eine Emulsion umfassend eine Fettphase und eine wässrige Phase umfasst.

25. Verfahren zur kosmetischen Behandlung von Anzeichen von Hautalterung, **dadurch gekennzeichnet, dass** es darin besteht, dass man eine Zusammensetzung nach einem der Ansprüche 22 bis 24 auf die Haut aufträgt.

26. Verwendung des Produkts nach einem der Ansprüche 1 bis 7 und 19 bis 21 in der Kosmetik zum Hydratisieren der oberen Hautschichten.

27. Produkt nach einem der Ansprüche 1 bis 7 und 19 bis 21 zur Verwendung als Arzneimittel.

28. Produkt nach einem der Ansprüche 1 bis 7 und 19 bis 21 zur Verwendung in der Behandlung von Erythemen von empfindlicher Haut oder Haut, die Reaktionen zeigt.

29. Produkt nach einem der Ansprüche 1 bis 7 und 19 bis 21 zur Verwendung im Schutz der Haut gegen Ultraviolettstrahlen.

30. Produkt nach einem der Ansprüche 1 bis 7 und 19 bis 21 zur Verwendung in der Behandlung von sonnenbedingten Erythemen.

## Claims

1. Cosmetic and/or dermatological product comprising:
0.5% to 10% by weight, preferably from 1% to 7% by weight of saturated or unsaturated, linear or branched, C₆₋₃₀, preferably C₆₋₂₄ fatty acids,
3% to 15% by weight, preferably from 5% to 10% by weight of saturated or unsaturated, linear or branched C₆₋₃₀ fatty alcohols,
0.5% to 10% by weight, preferably from 1% to 5% by weight of one or more phytosterols,
0.1% to 10% by weight, preferably from 0.5% to 5% by weight of one or more terpenes other than squalene,
1% to 15% by weight, preferably from 3% to 10% by weight of squalene, and
50% to 90% by weight, preferably from 55% to 85% by weight of triglycerides,
relative to the total weight of the product.

2. Cosmetic and/or dermatological product according to Claim 1, **characterized in that** the C₆₋₃₀ fatty acids are linear and saturated, monounsaturated or diunsaturated.

3. Cosmetic and/or dermatological product according to Claim 1 or 2, **characterized in that** the C₆₋₃₀ fatty acids are chosen from hexanoic (or caproic) acid, octanoic (or caprylic) acid, decanoic (or capric) acid, lauric acid, myristic acid, palmitic acid, margaric acid, stearic acid, arachidic acid, behenic acid, lignoceric acid, palmitoleic acid, oleic acid and linoleic acid, and mixtures thereof.

4. Cosmetic and/or dermatological product according to any one of the preceding claims, **characterized in that** that the C₆₋₃₀ fatty acids are in the form of a mixture of
0.2% to 1.5% by weight of hexanoic (or caproic) acid,
4% to 20% by weight of octanoic (or caprylic) acid,
2% to 15% by weight of decanoic (or capric) acid,
30% to 60% by weight of lauric acid,
10% to 30% by weight of myristic acid,
4% to 12% by weight of palmitic acid,
1% to 6% by weight of stearic acid,
1% to 11% by weight of oleic acid,
0.5% to 4% by weight of linoleic acid and
less than 3% by weight of C₂₀₋₃₀ fatty acid.

5. Cosmetic and/or dermatological product according to any one of the preceding claims, **characterized in that** the terpenes other than squalene are friedelin, betulin, lupen-3-one, sitost-4-en-3-one, and triterpene alcohols such as lupeol, betulinol, α-amyrin and erythrodiol.

6. Cosmetic and/or dermatological product according to any one of the preceding claims, **characterized in that** it contains more than 20% by weight, better still from 20% to 50% by weight, of terpene compounds including squalene, terpenes other than squalene and phytosterols.

7. Cosmetic and/or dermatological product according to any one of the preceding claims, **characterized in that** the triglycerides are esters of glycerol and of C₄₋₃₀, preferably C₄₋₂₄, fatty acids.

8. Process for preparing a cosmetic and/or dermatological product according to any one of Claims 1 to 7, from cork and from at least one portion of the tree that provides cork, chosen from its leaves, its flowers, its roots, its buds and mixtures thereof, preferably from its leaves, its flowers and mixtures thereof, comprising the steps consisting in:
a) bringing the cork into contact with supercritical CO₂, at a temperature ranging from 10 to 120°C and under a pressure ranging from 1 to 60 MPa (10 to 600 bar), optionally in the presence of a co-solvent,
b) recovering the cork extract entrained by the supercritical CO₂, in the form of a mixture of aqueous and fatty phases,
c) separating the fatty phase of the cork extract from the aqueous phase,
d) mixing said fatty phase of the cork extract with one or more portions of the tree that provides cork, reduced to powder, and one or more natural fatty substances, at a temperature above the melting points of the fatty phase and of the natural fatty substance(s), under an atmosphere free or essentially free of O₂,
e) heating the mixture obtained at a temperature ranging from 100 to 140°C, preferably from 110 to 130°C, for a period of less than 20 minutes, preferably less than 15 minutes, under an atmosphere free or essentially free of O₂,
f) carrying out a microdispersion of said fatty phase of the cork extract, of one or more portions of the tree that provides cork, reduced to powder, in the natural fatty substance(s), at a temperature above the melting points of the fatty phase and of the natural fatty substance(s), under an atmosphere free or essentially free of O₂, and in
g) optionally repeating the two steps e) and f).

9. Process according to Claim 8, **characterized in that** the co-solvent of step a) is chosen from water and mixtures of water and C₁₋₄ alcohol.

10. Process according to Claim 8 or 9, **characterized in that** the co-solvent of step a) is present in an amount ranging from 0.01% to 10% by weight, preferably from 0.02% to 1% by weight, relative to the weight of the supercritical CO₂ used.

11. Process according to any one of Claims 8 to 10, **characterized in that** the fatty phase of the cork extract obtained in step c) is treated, before step d), by heating with stirring at a temperature above the melting point of the fatty phase, under an atmosphere free or essentially free of O₂, preferably from 15 to 60 minutes.

12. Process according to Claim 11, **characterized in that** the heating temperature ranges from 40 to 90°C, preferably from 50 to 60°C.

13. Process according to any one of Claims 8 to 12, **characterized in that** the portion(s) of the tree that provides cork is or are firstly harvested, dehydrated in air, and dried at a temperature of less than or equal to 40°C so as to obtain a residual water content ranging from 5% to 15% by weight, preferably from 8% to 10% by weight, relative to the total weight of the mixture obtained, then secondly milled and pulverized at a temperature ranging from -18 to -80°C, before use thereof in step d).

14. Process according to any one of Claims 8 to 13, **characterized in that** the portion(s) of the tree that provides cork, reduced to powder, have a particle size ranging from 100 µm to 1 mm.

15. Process according to any one of Claims 8 to 14, **characterized in that** the natural fatty substance(s) is (are) chosen from rapeseed oil, sunflower oil, soya oil, olive oil, sesame oil, apricot kernel oil, grapeseed oil, sweet almond oil, safflower oil, hazelnut oil, argan oil, copra or coconut butter, cocoa butter, shea butter and mixtures thereof.

16. Process according to any one of Claims 8 to 15, **characterized in that** the portions of the tree that provides cork are its leaves and its flowers and the mixing in d) is carried out using:
20% to 40% by weight of fatty phase of the cork extract,
0.01% to 15% by weight of powdered leaves,
0.01% to 15% by weight of powdered flowers, and
50% to 80% of one or more natural fatty substance(s),
relative to the total weight of the mixture.

17. Process according to any one of Claims 8 to 16, **characterized in that**, between each step e) and f), a maceration step is carried out for more than one hour, preferably from 2 to 4 hours.

18. Process according to any one of Claims 8 to 17, **characterized in that** step f) is carried out for a period of less than 30 minutes, preferably ranging from 2 to 30 minutes.

19. Cosmetic and/or dermatological product **characterized in that** it can be obtained by means of the process according to any one of Claims 8 to 18.

20. Cosmetic and/or dermatological product according to any one of Claims 1 to 7, **characterized in that** it can be obtained by means of the processes claimed in any one of Claims 8 to 18.

21. Cosmetic and/or dermatological product according to Claim 19 or 20, which is in semi-solid form at a temperature of 20-25°C and which has a melting point of about 30°C.

22. Cosmetic and/or dermatological composition comprising the cosmetic and/or dermatological product according to any one of Claims 1 to 7 and 19 to 21 in a cosmetically and/or dermatologically acceptable medium.

23. Cosmetic and/or dermatological composition according to Claim 22, **characterized in that** the cosmetic and/or dermatological product is present in an amount ranging from 0.1% to 10% by weight, better still from 0.5% to 8% by weight, and preferentially from 1% to 5% by weight, relative to the total weight of the composition.

24. Cosmetic and/or dermatological composition according to Claim 22 or 23, **characterized in that** the cosmetically and/or dermatologically acceptable medium comprises a single fatty phase or an emulsion comprising a fatty phase and an aqueous phase.

25. Process for cosmetic treatment of the signs of skin ageing, **characterized in that** it consists in applying, to the skin, a composition according to any one of Claims 22 to 24.

26. Use of the product according to any one of Claims 1 to 7 and 19 to 21, in cosmetics, for moisturizing the upper layers of the skin.

27. Product according to any one of Claims 1 to 7 and 19 to 21, for use thereof as a medicament.

28. Product according to any one of Claims 1 to 7 and 19 to 21, for use thereof in the treatment of erythema of sensitive or reactive skin.

29. Product according to any one of Claims 1 to 7 and 19 to 21, for use thereof in the protection of the skin against ultraviolet rays.

30. Product according to any one of Claims 1 to 7 and 19 to 21, for use thereof in the treatment of solar erythema.
